# EUROPÄISCHE PATENTSCHRIFT

(11) **EP 4 260 808 B1**
(45) Veröffentlichungstag und Bekanntmachung des Hinweises auf die Patenterteilung: **11.03.2026**
(21) Anmeldenummer: 22182323.0
(22) Anmeldetag: 30.06.2022
(51) Int. Cl.: A61B 6/03, A61B 6/04, A61B 6/00

(54) **COMPUTERTOMOGRAPHIEGERÄT MIT EINER KÖRPERSTÜTZVORRICHTUNG**
COMPUTER TOMOGRAPHY UNIT WITH BODY SUPPORT
APPAREIL DE TOMOGRAPHIE ASSISTÉE PAR ORDINATEUR DOTÉ D'UN DISPOSITIF DE SUPPORT DE CORPS

(43) Veröffentlichungstag der Anmeldung: 18.10.2023
(73) Patentinhaber: Siemens Healthineers AG, 91301 Forchheim (DE)
(72) Erfinder: Fehre, Jens, 91353 Hausen (DE); Giebel, Steffen, 91369 Wiesenthau (DE); Herbolzheimer, Jens, 91058 Erlangen (DE); Köhler, Marco, 91325 Adelsdorf (DE); Ramsauer, Martin, 90602 Pyrbaum (DE)
(74) Vertreter: Siemens Healthineers Patent Attorneys

(56) Entgegenhaltungen:
- EP-B1- 2 600 770
- WO-A1-2020/106402
- DE-A1- 102020 206 784
- US-A1- 2008 005 844

## Beschreibung

Die Erfindung betrifft ein Computertomographiegerät mit einer Körperstützvorrichtung zur Stützung eines Körpers eines Menschen. Die Erfindung betrifft ferner ein medizinisches Bildgebungssystem und ein Verfahren zum Anordnen eines Bretts einer Körperstützvorrichtung relativ zu einer Gantry eines Computertomographiegeräts.

Bei einer Untersuchung, die mit Hilfe eines bildgebenden Geräts durchgeführt wird, kann es erforderlich sein, dass ein erster Körperabschnitt eines Menschen vorübergehend von dem bildgebenden Gerät getragen wird, während gleichzeitig ein zweiter Körperabschnitt des Menschen auf einem von dem bildgebenden Gerät separaten Patientenbett aufliegt. Beispielsweise kann bei einer Kopfbildgebung mittels eines Kopf-Computertomographiegeräts die Patientenlagerung derart zwischen dem Kopf-Computertomographiegerät und einem Patientenbett aufgeteilt werden, dass das Kopf-Computertomographiegerät einen ersten Körperabschnitt, der den Kopf und den Schulterbereich umfasst, trägt und das Patientenbett einen zweiten Körperabschnitt, der den Beckenbereich und die Beine umfasst, trägt. Um diese aufgeteilte Patientenlagerung sowie einen Wechsel zwischen der aufgeteilten Patientenlagerung und einer vollständig von dem Patientenbett getragenen Patientenlagerung sicher und stabil zu ermöglichen, kann eine entsprechende Körperstützvorrichtung verwendet werden. Sowohl das bildgebende Gerät als auch das Patientenbett kann jeweils mobil ausgeführt sein. Daher sollte eine solche Körperstützvorrichtung mit der Mobilität des bildgebenden Geräts und des Patientenbetts, insbesondere relativ zueinander, zumindest in denjenigen Abschnitten des klinischen Arbeitsablaufs, die eine solche Mobilität vorsehen, kompatibel sein.

Als Stand der Technik sind hierbei die DE 10 2020 206784 A1, WO 2020/106402 A1 und EP 2 600 770 B1 zu nennen.

Die Erfindung hat die Aufgabe, ein bildgebendes Gerät bereitzustellen, welches in Bezug auf eine Handhabung eines Bretts einer Körperstützvorrichtung für eine Patientenlagerung, die zwischen dem bildgebenden Gerät und einem Patientenbett aufgeteilt ist, verbessert ist. Jeder Gegenstand eines unabhängigen Anspruchs löst diese Aufgabe. In den abhängigen Ansprüchen sind weitere vorteilhafte Aspekte der Erfindung berücksichtigt.

Die Erfindung betrifft ein Computertomographiegerät, aufweisend eine Gantry mit einer Öffnung und eine Körperstützvorrichtung zur Stützung eines Körpers eines Menschen,
- wobei die Körperstützvorrichtung ein Brett aufweist und derart relativ zu der Öffnung angeordnet ist, dass ein Schulterbereich des Körpers des Menschen auf dem Brett aufliegt, wenn sich das Brett in einer Untersuchungsposition des Bretts relativ zu der Gantry befindet und ein Kopf des Menschen entlang einer Systemachse der Gantry in die Öffnung eingeführt ist,
- wobei die Körperstützvorrichtung eine Schwenkvorrichtung aufweist und das Brett mittels der Schwenkvorrichtung derart relativ zu der Gantry um eine Schwenkachse schwenkbar gelagert ist, dass durch eine erste Schwenkbewegung des Bretts um die Schwenkachse das Brett relativ zu der Gantry von einer Vorbereitungsposition des Bretts bis zu der Untersuchungsposition des Bretts bewegt werden kann,
- wobei sich das Brett (7) flächig in einer Brettebene (7E) erstreckt, wobei die Brettebene (7E) zu der Schwenkachse (7A) im Wesentlichen parallel ist,
- dadurch gekennzeichnet, dass die Brettebene (7E) zu der Systemachse (SA) der Gantry (20) im Wesentlichen senkrecht ist, wenn sich das Brett (7) in der Vorbereitungsposition des Bretts (7) befindet, dass die erste Schwenkbewegung des Bretts um die Schwenkachse ein Absenken des Bretts von der Vorbereitungsposition bis zu der Untersuchungsposition bewirkt, und dass die Körperstützvorrichtung ferner ein Arretiersystem aufweist, wobei das Arretiersystem zum Arretieren des Bretts in der Vorbereitungsposition des Bretts relativ zu der Gantry eingerichtet ist.

Das Computertomographiegerät kann insbesondere als Kopf-Computertomographiegerät und/oder als mobiles Computertomographiegerät ausgebildet sein.

Insbesondere kann vorgesehen sein, dass der Mensch entlang der Systemachse der Gantry relativ zu der Gantry bewegt werden kann, insbesondere durch medizinisches Personal getragen werden kann, um dadurch den Kopf des Menschen in die Öffnung einzuführen, wenn sich das Brett in der Untersuchungsposition des Bretts befindet.

Insbesondere kann vorgesehen sein, dass das Brett mittels der Schwenkvorrichtung derart relativ zu der Gantry um die Schwenkachse schwenkbar gelagert ist, dass durch eine zweite Schwenkbewegung des Bretts um die Schwenkachse das Brett relativ zu der Gantry von der Untersuchungsposition des Bretts bis zu der Vorbereitungsposition des Bretts bewegt werden kann. Insbesondere kann die zweite Schwenkbewegung des Bretts um die Schwenkachse ein Anheben des Bretts von der Untersuchungsposition bis zu der Vorbereitungsposition bewirken.

Das Brett kann insbesondere formstabil und/oder plattenförmig ausgebildet sein. Die Öffnung kann beispielsweise tunnelförmig sein und/oder sich entlang der Systemachse erstrecken. Die Systemachse kann beispielsweise durch die Öffnung hindurch verlaufen, insbesondere durch einen zentralen Bereich der Öffnung verlaufen. Die Systemachse kann beispielsweise durch ein Isozentrum des Computertomographiegeräts verlaufen. Die Schwenkvorrichtung kann insbesondere ein Schwenklager sein und/oder in Form eines Klappmechanismus ausgebildet sein.

Eine Ausführungsform sieht vor, dass das Arretiersystem zum Arretieren des Bretts in der Untersuchungsposition des Bretts relativ zu der Gantry eingerichtet ist.

Das Arretiersystem kann insbesondere dazu eingerichtet sein, das Brett formschlüssig und/oder kraftschlüssig gegen ein Anheben zu sichern, wenn sich das Brett in der Vorbereitungsposition befindet. Das Arretiersystem kann insbesondere dazu eingerichtet sein, das Brett formschlüssig und/oder kraftschlüssig gegen ein Absenken und/oder gegen ein Anheben zu sichern, wenn sich das Brett in der Untersuchungsposition befindet. Das Arretiersystem kann insbesondere dazu eingerichtet sein, das Brett formschlüssig und/oder kraftschlüssig gegen ein Absenken und/oder gegen ein Anheben zu sichern, wenn sich das Brett in der Transportposition befindet.

Eine Ausführungsform sieht vor, dass die Körperstützvorrichtung ferner einen Dämpfer zum Abbremsen der ersten Schwenkbewegung des Bretts relativ zu der Gantry aufweist. Der Dämpfer kann beispielsweise in die Schwenkvorrichtung integriert sein. Der Dämpfer kann beispielweise ein Rotationsdämpfer und/oder eine Drehfeder sein.

Eine Ausführungsform sieht vor, dass die Systemachse im Wesentlichen horizontal, insbesondere horizontal, ist und/oder dass die Schwenkachse im Wesentlichen horizontal, insbesondere horizontal, ist.

Eine Ausführungsform sieht vor, dass sich die Schwenkachse in Bezug auf eine vertikale Richtung unterhalb der Systemachse befindet. Insbesondere kann sich die Schwenkachse in Bezug auf die vertikale Richtung auf Höhe eines unteren Rands der Öffnung befinden.

Die Erfindung sieht vor, dass sich das Brett flächig in einer Brettebene erstreckt und dass die Brettebene zu der Schwenkachse im Wesentlichen parallel ist. Insbesondere kann vorgesehen sein, dass sich das Brett flächig zwischen einem bettseitigen Rand des Bretts und einem gantryseitigen Rand des Bretts erstreckt, insbesondere wenn sich das Brett in der Untersuchungsposition befindet. Insbesondere kann der gantryseitige Rand des Bretts an der Schwenkachse angeordnet sein. Der bettseitige Rand des Bretts kann sich insbesondere auf einer der Schwenkachse abgewandten Seite des Bretts befinden.

Die Erfindung sieht vor, dass die Brettebene zu der Systemachse im Wesentlichen senkrecht ist, wenn sich das Brett in der Vorbereitungsposition des Bretts befindet.

Eine Ausführungsform sieht vor, dass das Brett von der Öffnung wegragt, wenn sich das Brett in der Untersuchungsposition des Bretts befindet.

Die Erfindung sieht vor, dass die erste Schwenkbewegung des Bretts um die Schwenkachse ein Absenken des Bretts bewirkt, nämlich ein Absenken des Bretts von der Vorbereitungsposition bis zu der Untersuchungsposition bewirkt.

Weiterhin kann vorgesehen sein, dass das Brett mittels der Schwenkvorrichtung derart relativ zu der Gantry um die Schwenkachse schwenkbar gelagert ist, dass durch eine Vorbereitungs-Schwenkbewegung des Bretts um die Schwenkachse das Brett relativ zu der Gantry von einer Transportposition des Bretts bis zu der Vorbereitungsposition des Bretts bewegt werden kann, wobei die Vorbereitungs-Schwenkbewegung des Bretts um die Schwenkachs ein Anheben des Bretts, insbesondere ein Anheben des Bretts von der Transportposition bis zu der Vorbereitungsposition, bewirkt.

Weiterhin kann vorgesehen sein, dass die Gantry einen Innenbereich und eine Verkleidung zum Abgrenzen des Innenbereichs von einer Umgebung aufweist und dass das Brett an einem Bereich der Verkleidung anliegt, wenn sich das Brett in der Transportposition des Bretts relativ zu der Gantry befindet. Insbesondere kann vorgesehen sein, dass die Brettebene zu der Systemachse im Wesentlichen senkrecht ist, wenn sich das Brett in der Transportposition des Bretts relativ zu der Gantry befindet. Der Raumbedarf der Gantry kann dadurch verringert werden, insbesondere um eine Transportbewegung der Gantry durchzuführen.

Insbesondere kann vorgesehen sein, dass das Brett mittels der Schwenkvorrichtung derart relativ zu der Gantry um die Schwenkachse schwenkbar gelagert ist, dass durch eine dritte Schwenkbewegung des Bretts um die Schwenkachse das Brett relativ zu der Gantry von der Vorbereitungsposition des Bretts bis zu der Transportposition des Bretts bewegt werden kann. Insbesondere kann die dritte Schwenkbewegung des Bretts um die Schwenkachse ein Absenken des Bretts von der Vorbereitungsposition bis zu der Transportposition bewirken. Insbesondere kann der Dämpfer ferner zum Abbremsen der dritten Schwenkbewegung des Bretts relativ zu der Gantry eingerichtet sein.

Eine Ausführungsform sieht vor, dass die Körperstützvorrichtung ferner eine Haltevorrichtung aufweist, wobei die Schwenkvorrichtung mittels der Haltevorrichtung mit der Gantry verbunden ist, wobei das Brett mittels der Schwenkvorrichtung mit der Haltevorrichtung verbunden und relativ zu der Haltevorrichtung um die Schwenkachse schwenkbar gelagert ist, wobei die Haltevorrichtung dazu eingerichtet ist, einen Abstand zwischen der Schwenkachse und der Systemachse veränderbar einzustellen, insbesondere abgestuft oder stufenlos veränderbar einzustellen.

Die Haltevorrichtung kann insbesondere dazu eingerichtet sein, den Abstand zwischen der Schwenkachse und der Systemachse durch ein Einstellen einer Höhe, in der die Schwenkachse in Bezug auf die vertikale Richtung verläuft, veränderbar einzustellen, wobei die Höhe der Systemachse in Bezug auf die vertikale Richtung unverändert bleibt.

Dazu kann die Haltevorrichtung beispielsweise teleskopisch ausgeführt sein. Die Haltevorrichtung kann beispielsweise eine Arretiervorrichtung aufweisen, um den eingestellten Abstand zwischen der Schwenkachse und der Systemachse zu halten.

Insbesondere kann vorgesehen sein, dass die Haltevorrichtung relativ zu der Gantry während der ersten Schwenkbewegung des Bretts um die Schwenkachse ruht und/oder dass die Haltevorrichtung relativ zu der Gantry während der Vorbereitungs-Schwenkbewegung des Bretts um die Schwenkachse ruht. Insbesondere kann vorgesehen sein, dass die Haltevorrichtung relativ zu der Gantry während der zweiten Schwenkbewegung des Bretts um die Schwenkachse ruht und/oder dass die Haltevorrichtung relativ zu der Gantry während der dritten Schwenkbewegung des Bretts um die Schwenkachse ruht.

Eine Ausführungsform sieht vor, dass die Gantry einen ersten Gantryteil und einen zweiten Gantryteil aufweist, wobei der erste Gantryteil einen drehbar gelagerten Rotor mit einem Projektionsdatenakquisitionssystem aufweist, wobei der zweite Gantryteil zumindest einen Abschnitt der Öffnung aufweist, wobei das Brett mittels der Schwenkvorrichtung mit dem zweiten Gantryteil verbunden und relativ zu dem zweiten Gantryteil um die Schwenkachse schwenkbar gelagert ist.

Weiterhin kann vorgesehen sein, dass der erste Gantryteil relativ zu dem zweiten Gantryteil derart bewegbar gelagert ist, dass eine Translationsbewegung des ersten Gantryteils relativ zu dem zweiten Gantryteil ausgeführt werden kann, während gleichzeitig dazu der zweite Gantryteil relativ zu dem Kopf des Menschen ruht und die Körperstützvorrichtung relativ zu dem Kopf des Menschen und relativ zu dem zumindest einen Abschnitt der Öffnung ruht, wenn sich der Kopf des Menschen in der Öffnung befindet.

Insbesondere kann vorgesehen sein, dass der erste Gantryteil eine Drehlagerung und eine Tragstruktur aufweist und dass der Rotor mittels der Drehlagerung mit der Tragstruktur verbunden und relativ zu der Tragstruktur um die Systemachse drehbar gelagert ist.

Die Erfindung betrifft ferner ein medizinisches Bildgebungssystem, aufweisend das erfindungsgemäße Computertomographiegerät und ein Patientenbett zur Lagerung des Menschen, wobei das Patientenbett in einer Untersuchungsposition des Patientenbetts relativ zu der Gantry angeordnet werden kann.

Insbesondere kann vorgesehen sein, dass ein Beckenbereich des Menschen auf dem Patientenbett aufliegt und/oder dass ein Beinbereich des Menschen auf dem Patientenbett aufliegt, wenn sich das Patientenbett in der Untersuchungsposition des Patientenbetts relativ zu der Gantry befindet, sich das Brett in der Untersuchungsposition des Bretts relativ zu der Gantry befindet, der Schulterbereich des Körpers des Menschen auf dem Brett aufliegt und ein Kopf des Menschen entlang einer Systemachse der Gantry in die Öffnung eingeführt ist. Das Patientenbett kann insbesondere ein mobiles Intensivpflegebett sein und/oder eine Untersuchungsliege eines Schlaganfalleinsatz-Mobils (Mobile Stroke Unit) sein.

Eine Ausführungsform sieht vor, dass das Brett eine Lücke, die sich zwischen der Gantry und dem Patientenbett im Wesentlichen senkrecht zu der Systemachse erstreckt, überbrückt, wenn sich das Patientenbett in der Untersuchungsposition des Patientenbetts befindet und sich das Brett in der Untersuchungsposition des Bretts befindet.

Eine Ausführungsform sieht vor, dass die Körperstützvorrichtung derart relativ zu der Gantry angeordnet ist, dass ein bettseitiger Rand des Bretts auf dem Patientenbett aufliegt, wenn sich das Patientenbett in der Untersuchungsposition des Patientenbetts befindet und sich das Brett in der Untersuchungsposition des Bretts befindet.

Die Erfindung betrifft ferner ein Verfahren zum Anordnen eines Bretts einer Körperstützvorrichtung relativ zu einer Gantry eines erfindungsgemäßen Computertomographiegeräts, wobei durch die erste Schwenkbewegung des Bretts um die Schwenkachse das Brett relativ zu der Gantry von der Vorbereitungsposition des Bretts bis zu der Untersuchungsposition des Bretts bewegt wird.

Weiterhin kann vorgesehen sein, dass durch eine Vorbereitungs-Schwenkbewegung des Bretts um die Schwenkachse das Brett relativ zu der Gantry von einer Transportposition des Bretts bis zu der Vorbereitungsposition des Bretts bewegt wird. Die Vorbereitungs-Schwenkbewegung des Bretts um die Schwenkachse kann beispielsweise ein Anheben des Bretts bewirken, insbesondere ein Anheben des Bretts von der Transportposition des Bretts bis zu der Vorbereitungsposition des Bretts bewirken. Die erste Schwenkbewegung des Bretts um die Schwenkachse kann beispielsweise ein Absenken des Bretts bewirken, insbesondere ein Absenken des Bretts von der Vorbereitungsposition bis zu der Untersuchungsposition bewirken.

Jede der beschriebenen Schwenkbewegungen des Bretts um die Schwenkachse kann beispielsweise erfolgen, indem das Brett jeweils manuell um die Schwenkachse geschwenkt wird, insbesondere von medizinischem Personal manuell um die Schwenkachse geschwenkt wird, insbesondere manuell hochgeklappt bzw. heruntergeklappt wird.

Weiterhin kann vorgesehen sein, dass die Körperstützvorrichtung ferner einen Schwenkantrieb zum Antreiben der ersten Schwenkbewegung und/oder zum Antreiben der zweiten Schwenkbewegung aufweist. Der Schwenkantrieb kann beispielsweise dazu eingerichtet sein, ein Drehmoment in Bezug auf die Schwenkachse auf das Brett auszuüben.

Insbesondere kann vorgesehen sein, dass die Vorbereitungs-Schwenkbewegung des Bretts um die Schwenkachse erfolgt, während das Patientenbett von der Gantry derart ausreichend weit entfernt ist, dass es die Vorbereitungs-Schwenkbewegung des Bretts um die Schwenkachse nicht stört. Weiterhin kann vorgesehen sein, dass eine erste Translationsbewegung des Patientenbetts relativ zu der Gantry entlang der Systemachse erfolgt, um das Patientenbett in die Untersuchungsposition des Patientenbetts relativ zu der Gantry zu bringen, während sich das Brett in der Vorbereitungsposition des Bretts relativ zu der Gantry befindet. Weiterhin kann vorgesehen sein, dass die erste Schwenkbewegung des Bretts um die Schwenkachse erfolgt, während sich das Patientenbett in der Untersuchungsposition des Patientenbetts relativ zu der Gantry befindet, insbesondere derart, dass die erste Schwenkbewegung des Bretts um die Schwenkachse durch ein Auftreffen des bettseitigen Rands des Bretts auf das Patientenbett begrenzt ist.

Ein Herunterklappen des Bretts aus einer nach oben geneigten Vorbereitungsposition herunter auf das Patientenbett kann vorteilhaft ohne Störung durch das Patientenbett erfolgen, auch wenn das Patientenbett sich nah an der Gantry befindet.

Weiterhin kann vorgesehen sein, dass die zweite Schwenkbewegung des Bretts um die Schwenkachse erfolgt, während sich das Patientenbett in der Untersuchungsposition des Patientenbetts relativ zu der Gantry befindet. Weiterhin kann vorgesehen sein, dass eine zweite Translationsbewegung des Patientenbetts relativ zu der Gantry entlang der Systemachse erfolgt, um das Patientenbett von der Gantry zu entfernen, während sich das Brett in der Vorbereitungsposition des Bretts relativ zu der Gantry befindet. Insbesondere kann vorgesehen sein, dass die dritte Schwenkbewegung des Bretts um die Schwenkachse erfolgt, während das Patientenbett von der Gantry derart ausreichend weit entfernt ist, dass es die dritte Schwenkbewegung des Bretts um die Schwenkachse nicht stört.

Durch die erfindungsgemäße Lösung wird eine aufgeteilte Patientenlagerung stabil und sicher realisierbar, wobei patientenspezifische anatomische Besonderheiten und/oder Besonderheiten des Patientenbetts, insbesondere die Betthöhe betreffend, im Vergleich zu herkömmlichen Lösungen besser berücksichtigt werden können. Ferner kann für eine Korrektur der Untersuchungsposition des Patientenbetts relativ zu der Gantry das Brett um die Schwenkachse hochgeklappt und nach erfolgter Korrektur heruntergeklappt werden. Das ist für das medizinische Person weniger arbeitsaufwendig als das Brett von der Gantry vollständig loszulösen und nach der Korrektur wieder mit der Gantry zu verbinden.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Die Verwendung der unbestimmten Artikel "ein" bzw. "eine" schließt nicht aus, dass das betroffene Merkmal auch mehrfach vorhanden sein kann.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.

Die Fig. 1 zeigt ein Computertomographiegerät mit einer Gantry und einer Körperstützvorrichtung.

Die Figuren 2 bis 7 zeigen das Computertomographiegerät für verschiedene Positionen eines Bretts der Körperstützvorrichtung relativ zu der Gantry.

Die Fig. 8 zeigt ein medizinisches Bildgebungssystem mit einem Computertomographiegerät und einem Patientenbett.

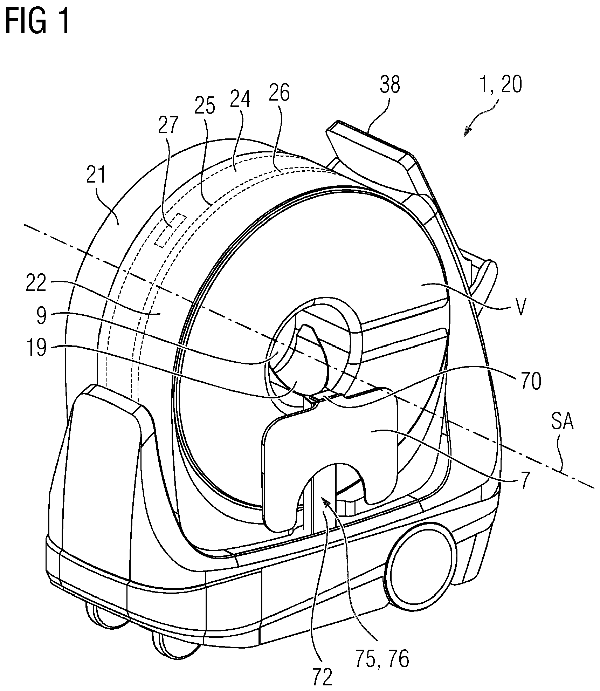

Die Fig. 1 zeigt ein Computertomographiegerät 1, aufweisend eine Gantry 20 mit einer Öffnung 9 und eine Körperstützvorrichtung 75 zur Stützung eines Körpers 15 eines Menschen 13.

Die Körperstützvorrichtung 75 weist ein Brett 7 auf ist und derart relativ zu der Öffnung 9 angeordnet, dass ein Schulterbereich des Körpers 15 des Menschen 13 auf dem Brett 7 aufliegt, wenn sich das Brett 7 in einer Untersuchungsposition des Bretts 7 relativ zu der Gantry 20 befindet und ein Kopf 14 des Menschen 13 entlang einer Systemachse SA der Gantry 20 in die Öffnung 9 eingeführt ist. Die Körperstützvorrichtung 75 weist eine Schwenkvorrichtung 70 auf und das Brett 7 ist mittels der Schwenkvorrichtung 70 derart relativ zu der Gantry 20 um eine Schwenkachse 7A schwenkbar gelagert, dass durch eine erste Schwenkbewegung des Bretts 7 um die Schwenkachse 7A das Brett 7 relativ zu der Gantry 20 von einer Vorbereitungsposition des Bretts 7 bis zu der Untersuchungsposition des Bretts 7 bewegt werden kann.

Die Körperstützvorrichtung 75 weist ferner ein Arretiersystem 76 auf, wobei das Arretiersystem 76 zum Arretieren des Bretts 7 in der Vorbereitungsposition des Bretts 7 relativ zu der Gantry 20 und/oder zum Arretieren des Bretts 7 in der Untersuchungsposition des Bretts 7 relativ zu der Gantry 20 eingerichtet ist. Die Körperstützvorrichtung 75 weist ferner einen in die Schwenkvorrichtung 70 integrierten Dämpfer zum Abbremsen der ersten Schwenkbewegung des Bretts 7 auf.

Die Körperstützvorrichtung 75 weist ferner eine Haltevorrichtung 72 auf, wobei die Schwenkvorrichtung 70 mittels der Haltevorrichtung 72 mit der Gantry 20 verbunden ist, wobei das Brett 7 mittels der Schwenkvorrichtung 70 mit der Haltevorrichtung 72 verbunden und relativ zu der Haltevorrichtung 72 um die Schwenkachse 7A schwenkbar gelagert ist, wobei die Haltevorrichtung 72 dazu eingerichtet ist, einen Abstand zwischen der Schwenkachse 7A und der Systemachse SA veränderbar einzustellen.

Die Gantry 20 weist einen ersten Gantryteil 21 und einen zweiten Gantryteil 22 auf, wobei der erste Gantryteil 21 einen drehbar gelagerten Rotor 24 mit einem Projektionsdatenakquisitionssystem 27 aufweist, wobei der zweite Gantryteil 22 zumindest einen Abschnitt der Öffnung 9 aufweist, wobei das Brett 7 mittels der Schwenkvorrichtung 70 mit dem zweiten Gantryteil 22 verbunden und relativ zu dem zweiten Gantryteil 22 um die Schwenkachse 7A schwenkbar gelagert ist.

Der erste Gantryteil 21 ist relativ zu dem zweiten Gantryteil 22 derart bewegbar gelagert, dass eine Translationsbewegung des ersten Gantryteils 21 relativ zu dem zweiten Gantryteil 22 ausgeführt werden kann, während gleichzeitig dazu der zweite Gantryteil 22 relativ zu dem Kopf 14 des Menschen 13 ruht und die Körperstützvorrichtung 75 relativ zu dem Kopf 14 des Menschen 13 und relativ zu dem zumindest einen Abschnitt der Öffnung 9 ruht, wenn sich der Kopf 14 des Menschen 13 in der Öffnung 9 befindet.

Der erste Gantryteil 21 weist eine Drehlagerung 25 und eine Tragstruktur 26 auf, wobei der Rotor 24 mittels der Drehlagerung 25 mit der Tragstruktur 26 verbunden und relativ zu der Tragstruktur 26 um die Systemachse SA drehbar gelagert ist.

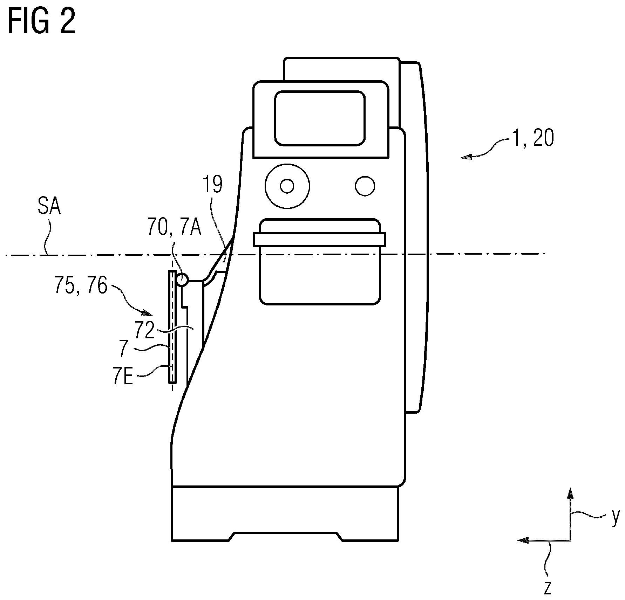

Die Figuren 2 bis 7 zeigen das Computertomographiegerät 1 für verschiedene Positionen eines Bretts 7 der Körperstützvorrichtung 75 relativ zu der Gantry 20.

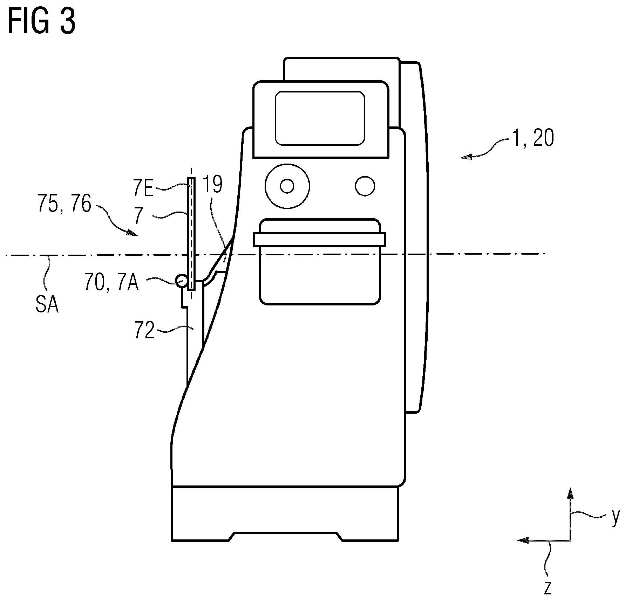

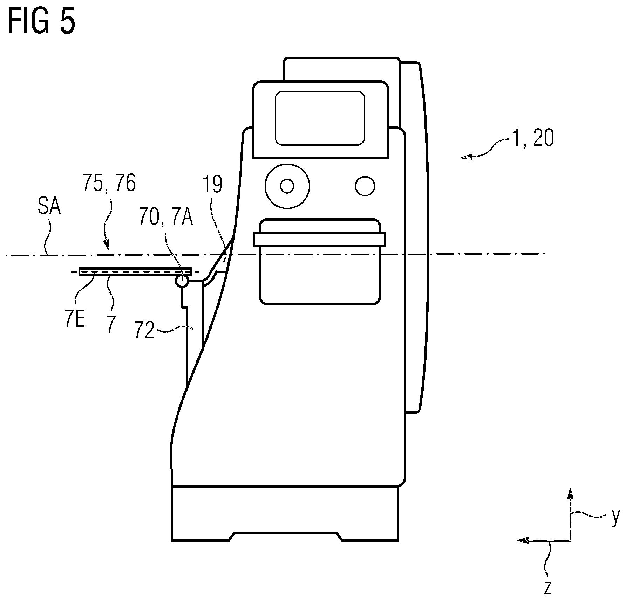

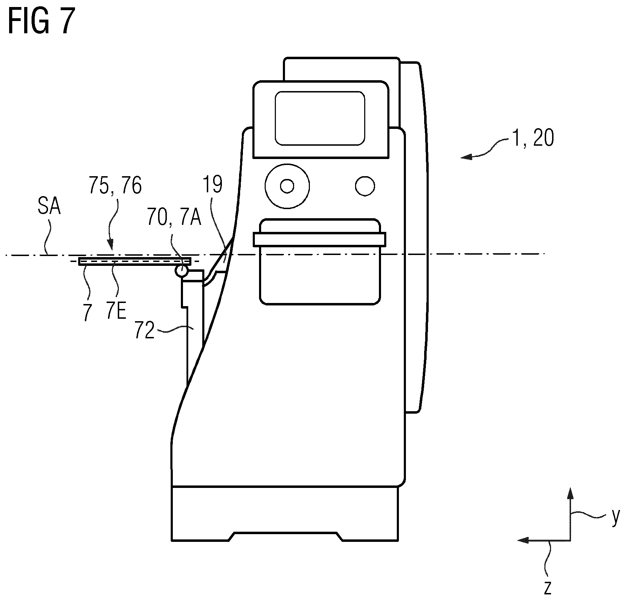

Die Systemachse SA ist horizontal und parallel zu der horizontalen Richtung z. Die Schwenkachse 7A ist horizontal und senkrecht zu der horizontalen Richtung z. Die Schwenkachse 7A befindet sich in Bezug auf die vertikale Richtung y unterhalb der Systemachse SA. Das Brett 7 erstreckt sich flächig in einer Brettebene 7E, wobei die Brettebene 7E zu der Schwenkachse 7A im Wesentlichen parallel ist. Die Brettebene 7E ist insbesondere dann zu der Systemachse SA im Wesentlichen senkrecht, wenn sich das Brett 7 in der Position gemäß Fig. 2 oder in der Position gemäß Fig. 3 befindet. Das Brett 7 ragt insbesondere dann von der Öffnung 9 weg, wenn sich das Brett 7 in der Position gemäß Fig. 5 oder in der Position gemäß Fig. 7 befindet.

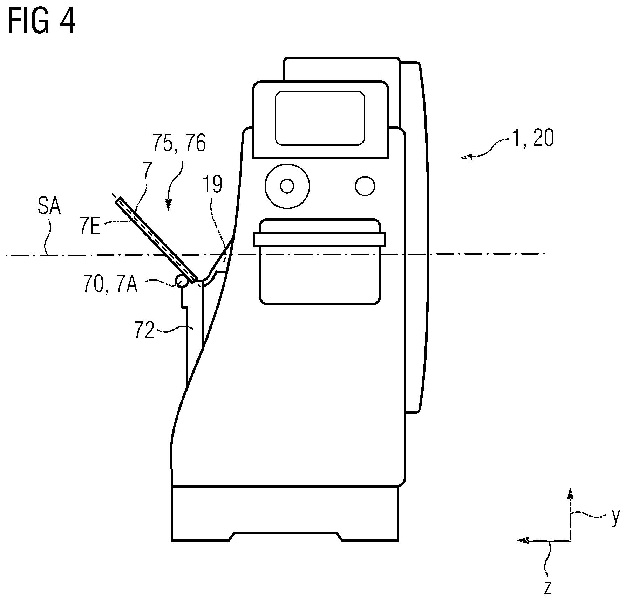

Die erste Schwenkbewegung des Bretts 7 um die Schwenkachse 7A bewirkt ein Absenken des Bretts 7, wenn durch die erste Schwenkbewegung des Bretts 7 um die Schwenkachse 7A das Brett 7 von der Position gemäß Fig. 3 bis zu der Position gemäß Fig. 5 bewegt wird oder wenn durch die erste Schwenkbewegung des Bretts 7 um die Schwenkachse 7A das Brett 7 von der Position gemäß Fig. 4 bis zu der Position gemäß Fig. 5 bewegt wird.

Erfindungsgemäß ist die in der Fig. 3 gezeigte Position des Bretts 7 die Vorbereitungsposition des Bretts 7. Gemäß einem Ausführungsbeispiel ist die in der Fig. 2 gezeigte Position des Bretts 7 die Transportposition des Bretts 7. und die in der Fig. 5 gezeigte Position des Bretts 7 die Untersuchungsposition des Bretts 7.

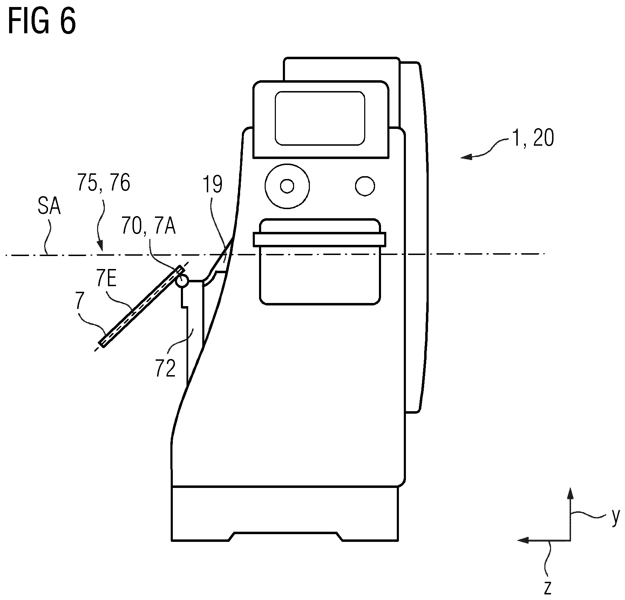

Gemäß einem weiteren Ausführungsbeispiel befindet sich die Untersuchungsposition des Bretts 7 zwischen der in der Fig. 5 gezeigten Position des Bretts 7 und der in der Fig. 6 gezeigten Position des Bretts 7.

In der Fig. 7 ist aufgrund einer Höhenverstellung mittels der Haltevorrichtung 72 der Abstand zwischen der Schwenkachse 7A und der Systemachse SA kleiner als in der Fig. 5. Durch die Höhenverstellung der Schwenkvorrichtung 70 hat sich die Höhe der Kopfschale nicht verändert. Gemäß einer anderen Ausführungsform ist eine Höhenverstellung der Kopfschale 19 relativ zu der Systemachse SA und/oder relativ zu der Schwenkachse 7A vorgesehen.

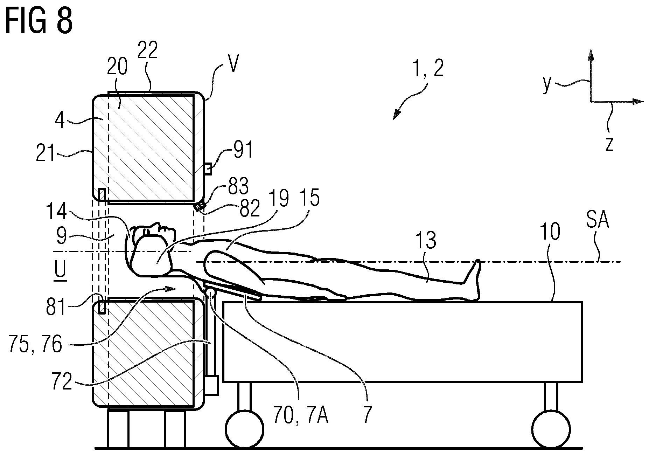

Die Fig. 8 zeigt ein medizinisches Bildgebungssystem 2, aufweisend das Computertomographiegerät 1 und ein Patientenbett 10 zur Lagerung des Menschen 13, wobei sich das Patientenbett 10 in einer Untersuchungsposition des Patientenbetts 10 relativ zu der Gantry 20 befindet und sich das Brett 7 in der Untersuchungsposition des Bretts 7 befindet. Das Computertomographiegerät 1 ist ein mobiles Kopf-Computertomographiegerät.

Das Computertomographiegerät 1 weist eine Kopfschale 19 auf, in welche der Kopf 14 des Menschen 13 aufgenommen ist.

Das Brett 7 überbrückt eine Lücke, die sich zwischen der Gantry 20 und dem Patientenbett 10 im Wesentlichen senkrecht zu der Systemachse SA erstreckt. Die Körperstützvorrichtung 75 ist derart relativ zu der Gantry 20 angeordnet, dass ein bettseitiger Rand des Bretts 7 auf dem Patientenbett 10 aufliegt. Das Brett 7 ruht relativ zu dem Patientenbett 10 auch dann, wenn die Translationsbewegung des ersten Gantryteils 21 relativ zu dem zweiten Gantryteil 22 ausgeführt wird.

Die Gantry 20 weist einen Innenbereich 4 und eine Verkleidung V zum Abgrenzen des Innenbereichs 4 von einer Umgebung U auf. Das Computertomographiegerät 1 weist einen Strahlenschutzkörper 81 auf, welcher mit der Gantry 20 derart lösbar verbunden ist, dass er eine Rückseite der Öffnung 9 abdeckt. Das Computertomographiegerät 1 weist eine Strahlenschutzvorrichtung 91 zur Abdeckung einer Vorderseite der Öffnung 9, beispielsweise mittels eines Strahlenschutzvorhangs, auf. Das Computertomographiegerät 1 weist ferner ein Kamerasystem 82 zur Patientenbeobachtung und/oder Patientenpositionierung und ein akustisches System 83 zur insbesondere bidirektionalen akustischen Patientenkommunikation auf.

## Patentansprüche

1. Computertomographiegerät (1), aufweisend eine Gantry (20) mit einer Öffnung (9) und eine Körperstützvorrichtung (75) zur Stützung eines Körpers (15) eines Menschen (13),
- wobei die Körperstützvorrichtung (75) ein Brett (7) aufweist und derart relativ zu der Öffnung (9) angeordnet ist, dass ein Schulterbereich des Körpers (15) des Menschen (13) auf dem Brett (7) aufliegt, wenn sich das Brett (7) in einer Untersuchungsposition des Bretts (7) relativ zu der Gantry (20) befindet und ein Kopf (14) des Menschen (13) entlang einer Systemachse (SA) der Gantry (20) in die Öffnung (9) eingeführt ist,
- wobei die Körperstützvorrichtung (75) eine Schwenkvorrichtung (70) aufweist und das Brett (7) mittels der Schwenkvorrichtung (70) derart relativ zu der Gantry (20) um eine Schwenkachse (7A) schwenkbar gelagert ist, dass durch eine erste Schwenkbewegung des Bretts (7) um die Schwenkachse (7A) das Brett (7) relativ zu der Gantry (20) von einer Vorbereitungsposition des Bretts (7) bis zu der Untersuchungsposition des Bretts (7) bewegt werden kann,
- wobei sich das Brett (7) flächig in einer Brettebene (7E) erstreckt, wobei die Brettebene (7E) zu der Schwenkachse (7A) im Wesentlichen parallel ist,
- **dadurch gekennzeichnet, dass** die Brettebene (7E) zu der Systemachse (SA) der Gantry (20) im Wesentlichen senkrecht ist, wenn sich das Brett (7) in der Vorbereitungsposition des Bretts (7) befindet, dass die erste Schwenkbewegung des Bretts (7) um die Schwenkachse (7A) ein Absenken des Bretts (7) von der Vorbereitungsposition bis zu der Untersuchungsposition bewirkt, und dass die Körperstützvorrichtung (75) ferner ein Arretiersystem (76) aufweist, wobei das Arretiersystem (76) zum Arretieren des Bretts (7) in der Vorbereitungsposition des Bretts (7) relativ zu der Gantry (20) eingerichtet ist.

2. Computertomographiegerät (1) nach Anspruch 1,
- wobei das Arretiersystem (76) zum Arretieren des Bretts (7) in der Untersuchungsposition des Bretts (7) relativ zu der Gantry (20) eingerichtet ist.

3. Computertomographiegerät (1) nach Anspruch 1 oder 2,
- wobei die Körperstützvorrichtung (75) ferner einen Dämpfer zum Abbremsen der ersten Schwenkbewegung des Bretts (7) aufweist.

4. Computertomographiegerät (1) nach einem der Ansprüche 1 bis 3,
- wobei die Systemachse (SA) im Wesentlichen horizontal ist und wobei die Schwenkachse (7A) im Wesentlichen horizontal ist.

5. Computertomographiegerät (1) nach einem der Ansprüche 1 bis 4,
- wobei sich die Schwenkachse (7A) in Bezug auf eine vertikale Richtung (y) unterhalb der Systemachse (SA) befindet.

6. Computertomographiegerät (1) nach einem der Ansprüche 1 bis 5,
- wobei das Brett (7) von der Öffnung (9) wegragt, wenn sich das Brett (7) in der Untersuchungsposition des Bretts (7) befindet.

7. Computertomographiegerät (1) nach einem der Ansprüche 1 bis 6,
- wobei das Arretiersystem (76) dazu eingerichtet ist, das Brett (7) formschlüssig und/oder kraftschlüssig gegen das Absenken zu sichern, wenn sich das Brett (7) in der Vorbereitungsposition befindet.

8. Computertomographiegerät (1) nach einem der Ansprüche 1 bis 7,
- wobei die Körperstützvorrichtung (75) ferner eine Haltevorrichtung (72) aufweist, wobei die Schwenkvorrichtung (70) mittels der Haltevorrichtung (72) mit der Gantry (20) verbunden ist, wobei das Brett (7) mittels der Schwenkvorrichtung (70) mit der Haltevorrichtung (72) verbunden und relativ zu der Haltevorrichtung (72) um die Schwenkachse (7A) schwenkbar gelagert ist,
- wobei die Haltevorrichtung (72) dazu eingerichtet ist, einen Abstand zwischen der Schwenkachse (7A) und der Systemachse (SA) veränderbar einzustellen.

9. Computertomographiegerät (1) nach einem der Ansprüche 1 bis 8,
- wobei die Gantry (20) einen ersten Gantryteil (21) und einen zweiten Gantryteil (22) aufweist,
- wobei der erste Gantryteil (21) einen drehbar gelagerten Rotor (24) mit einem Projektionsdatenakquisitionssystem (27) aufweist,
- wobei der zweite Gantryteil (22) zumindest einen Abschnitt der Öffnung (9) aufweist,
- wobei das Brett (7) mittels der Schwenkvorrichtung (70) mit dem zweiten Gantryteil (22) verbunden und relativ zu dem zweiten Gantryteil (22) um die Schwenkachse (7A) schwenkbar gelagert ist,
- wobei der erste Gantryteil (21) relativ zu dem zweiten Gantryteil (22) derart bewegbar gelagert ist, dass eine Translationsbewegung des ersten Gantryteils (21) relativ zu dem zweiten Gantryteil (22) ausgeführt werden kann, während gleichzeitig dazu der zweite Gantryteil (22) relativ zu dem Kopf (14) des Menschen (13) ruht und die Körperstützvorrichtung (75) relativ zu dem Kopf (14) des Menschen (13) und relativ zu dem zumindest einen Abschnitt der Öffnung (9) ruht, wenn sich der Kopf (14) des Menschen (13) in der Öffnung (9) befindet.

10. Medizinisches Bildgebungssystem (2), aufweisend das Computertomographiegerät (1) nach einem der Ansprüche 1 bis 9 und ein Patientenbett (10) zur Lagerung des Menschen (13), wobei das Patientenbett (10) in einer Untersuchungsposition des Patientenbetts (10) relativ zu der Gantry (20) angeordnet werden kann.

11. Medizinisches Bildgebungssystem (2) nach Anspruch 10,
- wobei das Brett (7) eine Lücke, die sich zwischen der Gantry (20) und dem Patientenbett (10) im Wesentlichen senkrecht zu der Systemachse (SA) erstreckt, überbrückt, wenn sich das Patientenbett (10) in der Untersuchungsposition des Patientenbetts (10) befindet und sich das Brett (7) in der Untersuchungsposition des Bretts (7) befindet.

12. Medizinisches Bildgebungssystem (2) nach Anspruch 10 oder 11,
- wobei die Körperstützvorrichtung (75) derart relativ zu der Gantry (20) angeordnet ist, dass ein bettseitiger Rand des Bretts (7) auf dem Patientenbett (10) aufliegt, wenn sich das Patientenbett (10) in der Untersuchungsposition des Patientenbetts (10) befindet und sich das Brett (7) in der Untersuchungsposition des Bretts (7) befindet.

13. Verfahren zum Anordnen eines Bretts (7) einer Körperstützvorrichtung (75) relativ zu einer Gantry (20) eines Computertomographiegeräts (1) nach einem der Ansprüche 1 bis 9,
- wobei durch die erste Schwenkbewegung des Bretts (7) um die Schwenkachse (7A) das Brett (7) relativ zu der Gantry (20) von der Vorbereitungsposition des Bretts (7) bis zu der Untersuchungsposition des Bretts (7) bewegt wird.

## Claims

1. Computed tomography device (1) having a gantry (20) with an opening (9) and a body support apparatus (75) for supporting a body (15) of a person (13),
- wherein the body support apparatus (75) has a board (7) and is arranged relative to the opening (9) such that a shoulder region of the body (15) of the person (13) rests on the board (7), when the board (7) is in an examination position of the board (7) relative to the gantry (20), and a head (14) of the person (13) is inserted into the opening (9) along a system axis (SA) of the gantry (20),
- wherein the body support apparatus (75) has a pivoting apparatus (70) and the board (7) is mounted pivotably about a pivot axis (7A) relative to the gantry (20) by means of the pivoting apparatus (70) such that a first pivoting movement of the board (7) about the pivot axis (7A) can move the board (7) relative to the gantry (20) from a preparation position of the board (7) to the examination position of the board (7),
- wherein the board (7) extends in a planar manner in a board plane (7E), wherein the board plane (7E) is substantially parallel to the pivot axis (7A),
- **characterised in that** the board plane (7E) is substantially perpendicular to the system axis (SA) of the gantry (20) when the board (7) is in the preparation position of the board (7), that the first pivoting movement of the board (7) about the pivot axis (7A) causes the board (7) to be lowered from the preparation position to the examination position, and that the body support apparatus (75) further has a locking system (76), wherein the locking system (76) is configured to lock the board (7) in the preparation position of the board (7) relative to the gantry (20).

2. Computed tomography device (1) according to claim 1,
- wherein the locking system (76) is configured to lock the board (7) in the examination position of the board (7) relative to the gantry (20).

3. Computed tomography device (1) according to claim 1 or 2,
- wherein the body support apparatus (75) further has a damper for braking the first pivoting movement of the board (7).

4. Computed tomography device (1) according to one of claims 1 to 3,
- wherein the system axis (SA) is substantially horizontal and wherein the pivot axis (7A) is substantially horizontal.

5. Computed tomography device (1) according to one of claims 1 to 4,
- wherein the pivot axis (7A) is located below the system axis (SA) with respect to a vertical direction (y).

6. Computed tomography device (1) according to one of claims 1 to 5,
- wherein the board (7) projects away from the opening (9) when the board (7) is in the examination position of the board (7).

7. Computed tomography device (1) according to one of claims 1 to 6,
- wherein the locking system (76) is configured to secure the board (7) in a positive-fitting and/or non-positive-fitting manner against lowering when the board (7) is in the preparation position.

8. Computed tomography device (1) according to one of claims 1 to 7,
- wherein the body support apparatus (75) further has a holding apparatus (72), wherein the pivoting apparatus (70) is connected to the gantry (20) by means of the holding apparatus (72), wherein the board (7) is connected to the holding apparatus (72) by means of the pivoting apparatus (70) and is mounted pivotably about the pivot axis (7A) relative to the holding apparatus (72),
- wherein the holding apparatus (72) is configured to set a distance between the pivot axis (7A) and the system axis (SA) in an adjustable manner.

9. Computed tomography device (1) according to one of claims 1 to 8,
- wherein the gantry (20) has a first gantry part (21) and a second gantry part (22),
- wherein the first gantry part (21) has a rotatably mounted rotor (24) with a projection data acquisition system (27),
- wherein the second gantry part (22) has at least one section of the opening (9),
- wherein the board (7) is connected to the second gantry part (22) by means of the pivoting apparatus (70) and is mounted pivotably about the pivot axis (7A) relative to the second gantry part (22),
- wherein the first gantry part (21) is mounted movably relative to the second gantry part (22) such that a translatory movement of the first gantry part (21) relative to the second gantry part (22) can be executed while at the same time the second gantry part (22) rests relative to the head (14) of the person (13) and the body support apparatus (75) rests relative to the head (14) of the person (13) and relative to the at least one section of the opening (9) when the head (14) of the person (13) is located in the opening (9).

10. Medical imaging system (2) having the computed tomography device (1) according to one of claims 1 to 9 and a patient bed (10) for supporting the person (13), wherein the patient bed (10) can be arranged in an examination position of the patient bed (10) relative to the gantry (20).

11. Medical imaging system (2) according to claim 10,
- wherein the board (7) bridges a gap extending between the gantry (20) and the patient bed (10) substantially perpendicular to the system axis (SA) when the patient bed (10) is in the examination position of the patient bed (10) and the board (7) is in the examination position of the board (7).

12. Medical imaging system (2) according to claim 10 or 11,
- wherein the body support apparatus (75) is arranged relative to the gantry (20) such that a bed-side edge of the board (7) rests on the patient bed (10) when the patient bed (10) is in the examination position of the patient bed (10) and the board (7) is in the examination position of the board (7).

13. Method for arranging a board (7) of a body support apparatus (75) relative to a gantry (20) of a computed tomography device (1) according to one of claims 1 to 9,
- wherein the first pivoting movement of the board (7) about the pivot axis (7A) moves the board (7) relative to the gantry (20) from the preparation position of the board (7) to the examination position of the board (7).

## Revendications

1. Appareil (1) de tomodensitométrie assisté par ordinateur comprenant un portique (20) ayant une ouverture (9) et un dispositif (75) d'appui du corps pour l'appui du corps (15) d'un homme (13),
- dans lequel le dispositif (75) d'appui du corps a une tablette (7) et est disposé par rapport à l'ouverture (9), de manière à ce qu'une zone d'épaule du corps (15) de l'homme (13) s'applique à la tablette (7), lorsque la tablette (7) se trouve en une position d'examen de la tablette (7) par rapport au portique (20) et lorsqu'une tête (14) de l'homme (13) est insérée dans l'ouverture (9) le long d'un axe (SA) de système du portique (20),
- dans lequel le dispositif (75) d'appui du corps a un dispositif (70) de pivotement et la tablette (7) est, au moyen du dispositif (70) de pivotement, montée pivotante autour d'un axe (7A) de pivotement par rapport au portique (20), de manière à ce que, par un premier mouvement de pivotement de la tablette (7) autour de l'axe (7A) de pivotement, la tablette (7) puisse être déplacée relativement au portique (20) d'une position de préparation de la tablette (7) à une position d'examen de la tablette (7),
- dans lequel la tablette (7) s'étend en surface dans un plan (7E) de tablette, dans lequel le plan (7E) de la tablette est sensiblement parallèle à l'axe (7A) de pivotement,
- **caractérisé en ce que** le plan (7E) de la tablette est sensiblement perpendiculaire à l'axe (SA) de système du portique (20), lorsque la tablette (7) se trouve dans la position de préparation de la tablette (7), **en ce que** le premier mouvement de pivotement de la tablette (7) autour de l'axe (7A) de pivotement provoque un abaissement de la tablette (7) de la position de préparation à la position d'examen, et **en ce que** le dispositif (75) d'appui du corps a en outre un système (76) d'arrêt, dans lequel le système (76) d'arrêt est agencé pour arrêter la tablette (7) dans la position de préparation de la tablette (7) par rapport au portique (20).

2. Appareil (1) de tomodensitométrie assisté par ordinateur suivant la revendication 1,
- dans lequel le système (76) d'arrêt est agencé pour arrêter la tablette (7) dans la position d'examen de la tablette (7) par rapport au portique (20).

3. Appareil (1) de tomodensitométrie assisté par ordinateur suivant la revendication 1 ou 2,
- dans lequel le dispositif (75) d'appui du corps a en outre un amortisseur pour freiner le premier mouvement de pivotement de la tablette (7).

4. Appareil (1) de tomodensitométrie assisté par ordinateur suivant la revendication 1 à 3,
- dans lequel l'axe (SA) de système est sensiblement horizontal et dans lequel l'axe (7A) de pivotement est sensiblement horizontal.

5. Appareil (1) de tomodensitométrie assisté par ordinateur suivant la revendication 1 à 4,
- dans lequel l'axe (7A) de pivotement se trouve, par rapport à une direction (y) verticale, en dessous de l'axe (SA) de système.

6. Appareil (1) de tomodensitométrie assisté par ordinateur suivant la revendication 1 à 5,
- dans lequel la tablette (7) sort de l'ouverture (9), lorsque la tablette (7) se trouve dans la position d'examen de la tablette (7).

7. Appareil (1) de tomodensitométrie assisté par ordinateur suivant l'une des revendications 1 à 6,
- dans lequel le système (76) d'arrêt est agencé, par complémentarité de forme et/ou coopération de force, pour empêcher la tablette (7) de s'abaisser, si la tablette (7) se trouve dans la position de préparation.

8. Appareil (1) de tomodensitométrie assisté par ordinateur suivant l'une des revendications 1 à 7,
- dans lequel le dispositif (75) d'appui du corps a en outre un dispositif (72) de maintien, dans lequel le dispositif (70) de pivotement est, au moyen du dispositif (72) de maintien, relié au portique (20), dans lequel la tablette (7) est reliée au dispositif (72) de maintien au moyen du dispositif (70) de pivotement et est montée à pivotement autour de l'axe (7A) de pivotement par rapport au dispositif (72) de maintien,
- dans lequel le dispositif (72) de maintien est agencé pour régler de manière variable une distance entre l'axe (7A) de pivotement et l'axe (SA) de système.

9. Appareil (1) de tomodensitométrie assisté par ordinateur suivant l'une des revendications 1 à 8,
- dans lequel le portique (20) a une première partie (21) de portique et une deuxième partie (22) de portique,
- dans lequel la première partie (21) de portique a un rotor (24) monté tournant et ayant un système (27) d'acquisition de données de projection,
- dans lequel la deuxième partie (22) du portique a au moins un segment de l'ouverture (9),
- dans lequel la tablette (7) est reliée à la deuxième partie (22) du portique au moyen du dispositif (70) de pivotement et est montée pivotante autour de l'axe (7A) de pivotement par rapport à la deuxième partie (22) de portique,
- dans lequel la première partie (21) de portique est montée mobile par rapport à la deuxième partie (22) de portique, de manière à pouvoir exécuter un mouvement de translation de la première partie (21) du portique par rapport à la deuxième partie (22) du portique, alors qu'en même temps la deuxième partie (22) du portique est au repos par rapport à la tête (14) de l'homme (13) et le dispositif (75) d'appui du corps est au repos par rapport à la tête (14) de l'homme (13) et par rapport au au moins un segment de l'ouverture (9), si la tête (14) de l'homme (13) se trouve dans l'ouverture (9).

10. Système (2) d'imagerie médicale, comportant l'appareil (1) de tomodensitométrie assisté par ordinateur suivant l'une des revendications 1 à 9 et une couchette (10) de patient pour coucher le patient (13), dans lequel la couchette (10) de patient peut être mise dans une position d'examen de la couchette (10) de patient par rapport au portique (20).

11. Système (2) d'imagerie médicale suivant la revendication 10,
- dans lequel la tablette (7) recouvre une lacune, qui s'étend sensiblement perpendiculairement à l'axe (SA) du système entre le portique (20) et la couchette (10) du patient, lorsque la couchette (10) de patient se trouve dans la position d'examen de la couchette (10) de patient et lorsque la tablette (7) se trouve dans la position d'examen de la tablette (7).

12. Système (2) d'imagerie médicale suivant la revendication 10 ou 11,
- dans lequel le dispositif (75) d'appui du corps est disposé par rapport au portique (20), de manière en ce qu'un bord, du côté de la couchette, de la tablette (7) s'applique à la couchette (10) de patient, lorsque la couchette (10) de patient se trouve dans la position d'examen de la couchette (10) de patient et lorsque la tablette (7) se trouve dans la position d'examen de la tablette (7).

13. Procédé pour disposer une tablette (7) d'un dispositif (75) d'appui du corps par rapport à un portique (20) d'un appareil (1) de tomodensitométrie assisté par ordinateur suivant l'une des revendications 1 à 9,
- dans lequel par le premier mouvement de pivotement de la tablette (7) autour de l'axe (7A) de pivotement, la tablette (7) est déplacée par rapport au portique (20) de la position de préparation de la tablette (7) à la position d'examen de la tablette (7).
